(19)

**Europäisches Patentamt**
**European Patent Office**
**Office européen des brevets**

(11) **EP 4 534 696 A1**

(12) **EUROPEAN PATENT APPLICATION**
published in accordance with Art. 153(4) EPC

(43) Date of publication:
**09.04.2025 Bulletin 2025/15**

(21) Application number: **23811228.8**

(22) Date of filing: **26.05.2023**

(51) International Patent Classification (IPC):
***C12Q 1/6886*** *(2018.01)*

(52) Cooperative Patent Classification (CPC):
**C12Q 1/6886**

(86) International application number:
**PCT/ES2023/070341**

(87) International publication number:
**WO 2023/227818 (30.11.2023 Gazette 2023/48)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.05.2022 ES 202230447**

(71) Applicants:
• **Servicio Andaluz de Salud**
**41071 Sevilla (ES)**
• **Universidad De Malaga**
**E-29071 Málaga (ES)**
• **Consorcio Centro de Investigación Biomédica en
Red**
**28029 Madrid (ES)**

(72) Inventors:
• **BARRAGÁN MALLOFRET, María Isabel**
**41071 Sevilla (ES)**
• **OLIVER MARTOS, Francisco Javier**
**41071 Sevilla (ES)**
• **ONIEVA ZAFRA, Juan Luis**
**41071 Sevilla (ES)**
• **RUEDA DOMÍNGUEZ, Antonio**
**41071 Sevilla (ES)**
• **PÉREZ RUIZ, Elisabeth**
**41071 Sevilla (ES)**
• **BERCIANO GUERRERO, Miguel Ángel**
**41071 Sevilla (ES)**
• **ALBA CONEJO, Emilio**
**41071 Sevilla (ES)**

(74) Representative: **San Martin Alarcia, Esther**
**C/ Zumaia 33A, 1º b**
**48007 Bilbao (ES)**

(54) **CIRCRNA PROFILE FOR PREDICTING IMMUNOTHERAPY RESPONSE IN CANCER PATIENTS**

(57) Disclosed are biomarkers, *in vitro* methods, and kits or devices, for predicting immunotherapy response using anti-PD1 and/or anti-PD-L1 immune checkpoint inhibitors and the clinical benefit for a human subject with metastatic cutaneous melanoma.

EP 4 534 696 A1

Processed by Luminess, 75001 PARIS (FR)

Description

## TECHNICAL FIELD

The present invention is framed in the field of medicine, specifically in precision immunotherapy against cancer and more specifically in the treatment of metastatic cutaneous melanoma. It concerns a set of functional biomarkers for response to anti-PD1 therapy that can be used for therapeutic decision-making within a strategy to predict treatment response and patient survival in terms of overall survival and disease-free progression.

## BACKGROUND OF THE INVENTION

Melanoma is an aggressive malignant tumor of epidermal melanocytes. Cutaneous melanoma (CM) is a common cancer with increasing incidence rates in the Western world and is the most lethal form of skin cancer. In 2040, 510,000 new cases are expected to be diagnosed and about 96,000 deaths. If cutaneous melanoma becomes metastatic, treatment options and chances of survival decrease dramatically. Immunotherapy treatments based on immune checkpoint inhibitors (ICBs), such as PD-1 and CTLA4, have been a breakthrough in the treatment of metastatic cutaneous melanoma and have changed the landscape of treatment options for cutaneous melanoma in recent years. Although a very promising therapy, primary resistance to immune checkpoint blockade arises in approximately 70% of patients with cutaneous melanoma treated with a CTLA-4 inhibitor and in 40-65% of patients with cutaneous melanoma who were given PD-1-targeted therapy (Aldea *et al.,* 2021)

CircRNAs can act as competitive endogenous RNAs (ceRNAs). (Tay, Rinn and Pandolfi, 2014) and give rise to an additional new post-transcriptional layer. The ceRNA hypothesis holds that biological processes are regulated by an intrinsic mechanism. It is becoming increasingly clear that circRNA deregulation is involved in carcinogenesis and progression of numerous cancers, acting as oncogenes or tumor suppressors (Montico *et al.,* 2021).. There is currently a great effort trying to determine reliable biomarkers to predict response to immunotherapy. PDL-L1, microsatellite instability and tumor mutational burden (TMB), among others, have been used. Of these, so far only tumor mutational burden (TMB) has been used in therapeutic trials, but it has not been found to be beneficial in melanoma patients, due to the high mutation rate of melanoma tumors (Filipovic, Miller and Bolen, 2020; Aldea et al., 2021). Most of the immune checkpoint-related studies performed to date have studied the biological significance of tumor infiltrating lymphocytes (TILs) focusing on T cells and tumor neoantigens.

## BRIEF DESCRIPTION OF THE INVENTION

The present invention addresses the lack of definitive biomarkers of immune checkpoint response or ICB in patients with metastatic melanoma by studying the differential transcript expression of responders versus non-responders to nivolumab using a transcriptomic approach involving circRNA transcripts. Thus, the potential of circRNA biomarkers for patient selection and adequacy of therapy in cancer patients, especially with cutaneous melanoma, is demonstrated.

Circular transcriptome analysis of large tumor samples from melanoma patients treated with the anti-PD1 agent nivolumab has been used to identify a signature of 23 circRNA genes associated with response. RNA-Seq is employed in the anti-PD1 therapy discovery cohort to select loci that might have genetic variants relevant to the resistance phenotype.

The expression of all loci related to the responder phenotype is also associated with overall survival ("OS") and disease-free progression ("PFS"), forming a transcriptomic signature of the response capable of predicting the overall survival (OS) of patients with metastatic melanoma treated with anti-PD1. Likewise, some of the 23 loci, specifically 13, present this prognostic value individually.

The initial cohort is based on 12 biopsies of metastatic cutaneous melanoma. Among these 12 patients there are 8 responders and 4 non-responders to treatment with anti-PD1 immunotherapy. Response or non-response criteria include:

- Non-responder: progression in less than 3 months from the start of immunotherapy.

  - Severe: patients not responding to treatment who present hyperprogression after treatment with immunotherapy.
  - Non-severe: those non-responders who previously had a poor prognosis with respect to immunotherapy treatment due to some adverse symptoms such as brain metastases or an "animal-like" tumor, so the lack of response to immunotherapy may not be related to the treatment but to the patient's tumor profile.

- Responder: patients with partial response ("PR"), complete response ("CR") or stable disease ("SD") for approximately one year or at least who have been on treatment for one year, following the partial and complete response criteria in the RECIST v1.1 ("Response Evaluation Criteria in Solid Tumors") solid tumor response criteria evaluation guideline.

In the present invention the term "responder" refers to those patients who have a complete or partial response to the drug, stable disease or have been on treatment for at least one year and with the term "non-responder" to those who do not respond to the same so that the tumor continues to progress in the short term (in less than 3 months) after the initiation of immunotherapeutic treatment.

The present invention relates to the use of the 23 loci described in Table 1, individually or in any of their combinations, to prognosticate or predict the response to anti-PD1 treatment of a human subject suffering from cancer. In a preferred embodiment, the human subject has metastatic cutaneous melanoma.

Table 1. List of circRNA loci differentially expressed between good and poor responders for patients with Metastatic Cutaneous Melanoma (MCM).

| Locus | Type | Good responder status |
|---|---|---|
| hsa-ALDH1L2_0014 | circRNA | Overexpressed |
| hsa-CC38_0001 | circRNA | Overexpressed |
| hsa-CD74_0005 | circRNA | Overexpressed |
| hsa-CDR1_0001 | circRNA | Underexpressed |
| hsa-CPM_0002 | circRNA | Overexpressed |
| hsa-ERBB3_0002 | circRNA | Overexpressed |
| hsa-HLA-DRB1_0004 | circRNA | Overexpressed |
| hsa-HLA-DRB1_0005 | circRNA | Overexpressed |
| hsa_IFI30_0001 | circRNA | Overexpressed |
| hsa-ITGA6_0030 | circRNA | Overexpressed |
| hsa-MS4A1_0002 | circRNA | Overexpressed |
| hsa-MX1_0002 | circRNA | Overexpressed |
| hsa-NRCAM_0112 | circRNA | Overexpressed |
| hsa-PARP14_0007 | circRNA | Overexpressed |
| hsa-PMP22_0004 | circRNA | Overexpressed |
| hsa-PTPRC_0003 | circRNA | Overexpressed |
| hsa-RP11-219A15_0001 | circRNA | Overexpressed |
| hsa-SLC6A6_0027 | circRNA | Overexpressed |
| hsa-SLIT2 0013 | circRNA | Underexpressed |
| hsa-SOX6_0020 | circRNA | Overexpressed |
| hsa-SYTL2_0003 | circRNA | Overexpressed |
| hsa-TIM22_0002 | circRNA | Overexpressed |
| hsa-UTRN_0056 | circRNA | Overexpressed |

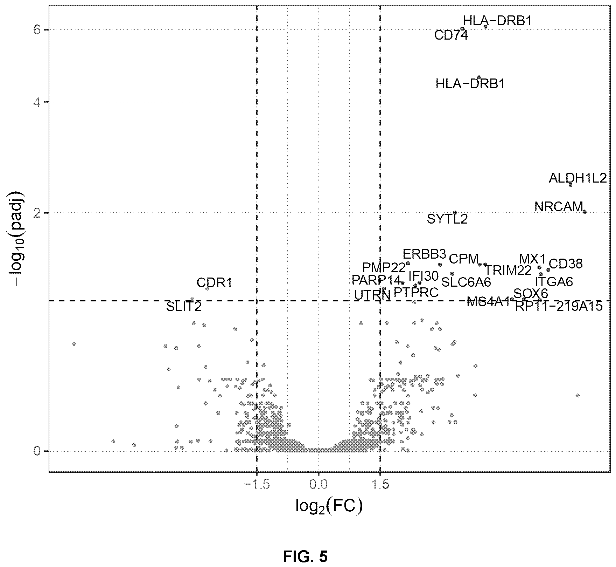

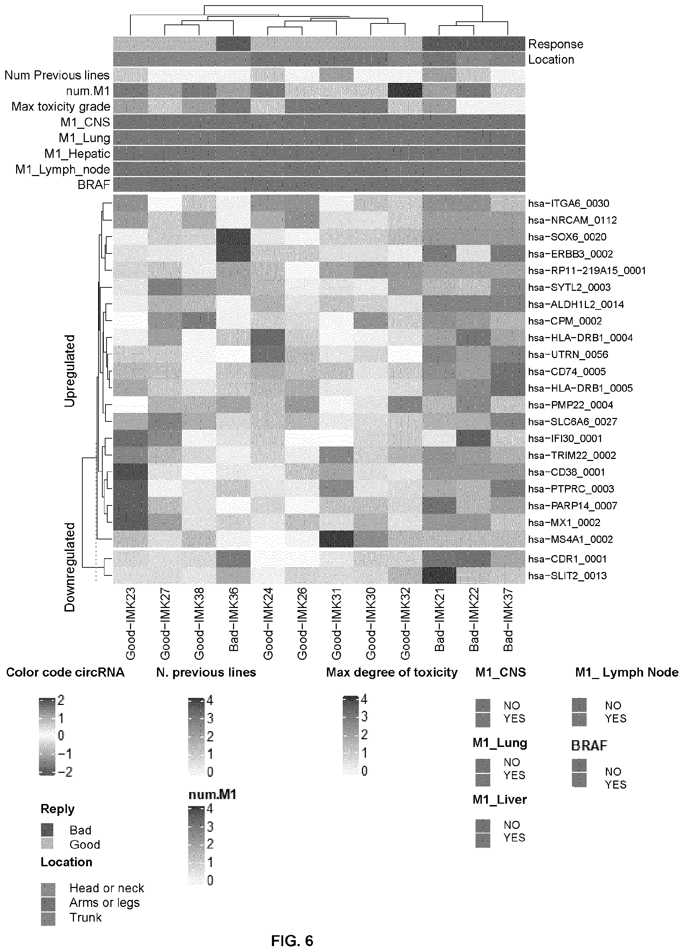

The 23 genes listed in Table 1 are differentially expressed between responders and non-responders to anti-PD1 treatment. Therefore, they are associated with response to anti-PD1 treatment and are biomarkers of response to nivolumab in metastatic cutaneous melanoma. Each of the 23 circular genes in Table 1 is individually associated with response to anti-PD1 treatment, as can be seen in Figures 5 and 6, which show the volcano plot and heatmap, indicators of the significant difference in the expression of these 23 genes in good responders to nivolumab.

Thus, another aspect of the invention refers to an expression profile of the 23 loci described in Table 1, both individually and in any of their combinations, as markers of response to anti-PD1 treatment, and thus as biomarkers of response to nivolumab in metastatic cutaneous melanoma.

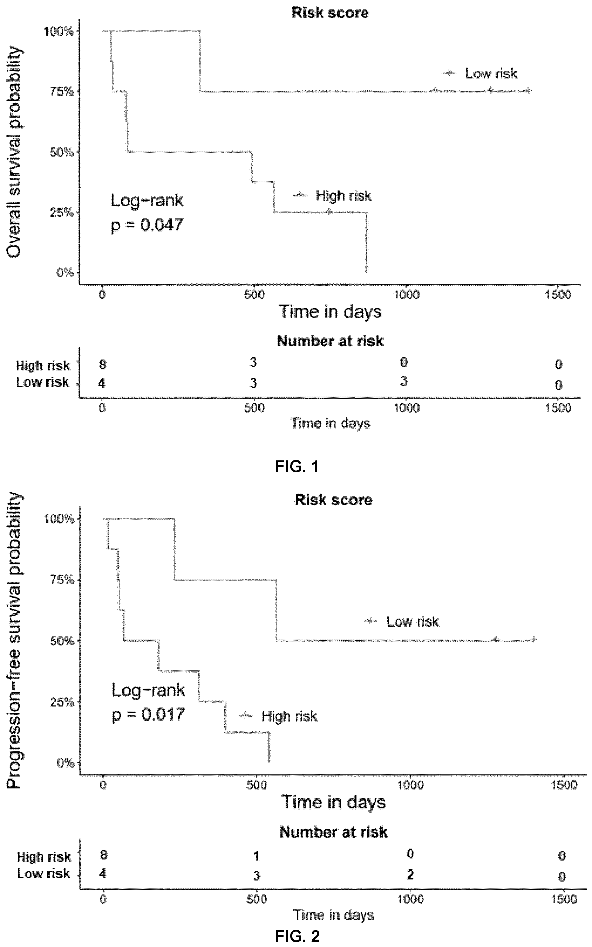

In addition, the overall survival (OS) and disease-free progression (FPS) score of the 23 genes as a whole also serves to predict patient survival. The signature as a whole is associated with OS with a p-value of 0.047 (Figure 1), and with FPS with a p-value of 0.017 (Figure 2), making it a biomarker of disease prognosis in patients with metastatic cutaneous melanoma treated with nivolumab.

Thus, in another aspect of the invention refers to a simultaneous form expression profile of the 23 loci described in Table 1 as prognostic markers of metastatic cutaneous melanoma in human subjects.

Reproduced below in Table 2 are all the loci in Table 1 to indicate the two parameters of clinical benefit used in this invention, OS and PFS, for each of the loci. Some genes are biomarkers for both, and others for one or the other. The p-values and Hazard Ratio (HR) of the individual genes are noted.

Table 2: List of the 23 circRNA loci in Table 1 noting their ability as prognostic markers of metastatic cutaneous melanoma in human subjects, measured as overall survival (OS) and/or disease-free progression (FPS).

| Locus | p-value logrank OS | Median OS (Low expressio n vs. High expressio n) | HR OS | p-value logran k PFS | Median PFS (Low expressio n vs High expressio n) | HRPFS | Locus | p-value logran k OS |
|---|---|---|---|---|---|---|---|---|
| hsa-ALDH1L2_00 14 | 0,6047632 56 | 526.5 [77;NR] vs. 321 [321;NR] | 0.578 (0.0707 4.7282) | 0,6092 | 0,3858655 96 | 354 [52;NR] vs. 205.5 [180;NR] | 2.0933 (0.3797 - 11.5397 ) | 0,3964 |
| hsa-CD38 0001 | 0,4555498 05 | 405.5 [77;NR] vs 563 [563;NR] | 0.4576 (0.0558 - 3.7555) | 0,4667 | 0,1137717 45 | 205.5 [52; NR] vs. 563 [563;NR] | 0.2048 (0.0243 - 1.7271) | 0,1449 |
| **hsa-CD74_0005** | 0,0383908 3 | 35 [NR;NR] vs 563 [321;NR] | 0.0953 (0.006 - 1.5255) | 0,0966 | 0,2973076 32 | 66 [NR;NR] vs. 311 [180;NR] | 0.3191 (0.0331 - 3.0772) | 0,3232 |
| **hsa-CDR1_0001** | 0,0115793 37 | 56 [35;NR] vs. 716 [321;NR] | 0.0839 (0.0074 - 0.9501) | 0,0454 | 0,0369014 6 | 57 [48;NR] vs. 354 [180;NR] | 0.1576 (0.0217 - 1.1433) | 0,0676 |
| hsa-CPM_0002 | 0,8261761 74 | 322 [77;NR] vs 679.5 [321;NR] | 0.8509 (0.201 - 3.6014) | 0,8264 | 0,5979829 42 | 231.5 [52;NR] vs. 271 [180;NR] | 1.4318 (0.3747 - 5.4721) | 0,5998 |
| **hsa-ERBB3_000 2** | 0,0167101 87 | 58 [28;NR] vs. 869 [490;NR] | 0.1862 (0.0404 - 0.8572) | 0,0309 | 0,2104221 66 | 59 [16;NR] vs. 354 [231;NR] | 0.4471 (0.1229 - 1.6262) | 0,2217 |
| **hsa-HLA-DRB1_0004** | 0,0011419 88 | 35 [28;NR] vs. 869 [490;NR] | 0.0568 (0.0057 - 0.5675) | 0,0146 | 0,0034332 89 | 52 [16;NR] vs 397 [231;NR] | 0.0709 (0.0072 - 0.7017) | 0,0236 |
| **hsa-HLA-DRB1_0005** | 0,0011419 88 | 35 [28;NR] vs. 869 [490;NR] | 0.0568 (0.0057 - 0.5675) | 0,0146 | 0,0034332 89 | 52 [16;NR] vs 397 [231;NR] | 0.0709 (0.0072 - 0.7017) | 0,0236 |
| **hsa-IFI30_0001** | 0,0009111 19 | 28 [NR;NR] vs 563 [321;NR] | 0.01 (0 -0.11) | 0,001 | 0,0009111 19 | 16 [NR;NR] vs. 311 [180;NR] | 0.01 (0 - 0.05) | 0,001 |
| hsa-ITGA6_0030 | 0,6047632 56 | 526.5 [77;NR] vs. 321 [321;NR] | 0.578 (0.0707 4.7282) | 0,6092 | 0,3381522 33 | 245.5 [52;NR] vs 231 [231;NR] | 0.377 (0.0474 3.0013) | 0,3567 |

EP 4 534 696 A1

(continued)

| Locus | p-value logrank OS | Median OS (Low expressio n vs. High expressio n) | HR OS | p-value logran k PFS | Median PFS (Low expressio n vs High expressio n) | HRPFS | Locus | p-value logran k OS |
|---|---|---|---|---|---|---|---|---|
| hsa-MS4A1_0002 | 0,6233252 | 595 [77;NR] vs 526.5 [490;NR] | 1.5079 (0.2895 7.8536) | 0,6257 | 0,7527615 83 | 205.5 [52; NR] vs 437 [311;NR] | 0.7786 (0.1634 3.7104) | 0,7534 |
| hsa-MX1_0002 | 0,2463268 87 | 201 [77;NR] vs 563 [490;NR]. | 0.3971 (0.079 - 1.9953) | 0,2622 | 0,3210080 63 | 148.5 [52;NR] vs 437 [180;NR] | 0.5048 (0.1277 - 1.9948) | 0,3295 |
| **hsa-NRCAM_011 2** | 0,2088293 71 | 285.5 [77;NR] vs. 869 [321;NR] | 0.3596 (0.0689 - 1.8779) | 0,2252 | 0,0405655 92 | 123 [52;NR] vs 539 [231;NR] vs 539 [231;NR] | 0.2161 (0.0441 - 1.0586) | 0,0588 |
| **hsa-PARP14_000 7** | 0,0369014 6 | 58 [35;NR] vs. 716 [321;NR] | 0.1576 (0.0217 - 1.1433) | 0,0676 | 0,0697824 1 | 59 [52;NR] vs. 354 [180;NR] | 0.1933 (0.0268 - 1.3954) | 0,1032 |
| hsa-PMP22_0004 | 0,1471015 07 | 58 [28;NR] vs. 716 [490;NR] | 0.339 (0.0736 - 1.5624) | 0,1653 | 0,0527583 33 | 59 [16;NR] vs 425 [231;NR] | 0.2689 (0.0652 - 1.1088) | 0,0692 |
| **hsa- PTPRC_0003** | 0,0113279 33 | 77 [35;NR] vs NR [490;NR] | 0.1775 (0.0405 - 0.779) | 0,022 | 0,0250229 6 | 52 [48;NR] vs 397 [231;NR] | 0.2362 (0.0608 - 0.9166) | 0,037 |
| hsa- RP11-219A15_000 1 | 0,5435337 16 | 716 [81;NR] vs 405.5 [77;NR] | 1.5951 (0.3491 - 7.2891) | 0,547 | 0,3133289 61 | 359.5 [66;NR] vs 271 [48;NR] | 2.0271 (0.4995 - 8.2272) | 0,3228 |
| hsa-SLC6A6_002 7 | 0,2088293 71 | 285.5 [77;NR] vs. 869 [321;NR] | 0.3596 (0.0689 - 1.8779) | 0,2252 | 0,5006962 91 | 188.5 [52;NR] vs. 385 [180;NR]. | 0.6288 (0.1611 - 2.4546) | 0,5044 |
| hsa-SLIT2 0013 | 0,6770310 8 | 563 [321;NR] vs. 81 [28;NR] | 1.4064 (0.2804 - 7.0535) | 0,6785 | 0,9747544 68 | 311 [180;NR] vs 52 [16;NR] | 1.0257 (0.2133 - 4.9313) | 0,9748 |
| **hsa-SOX6 0020** | 0,1458551 02 | 563 [321;NR] vs. 77 [NR;NR] | 4.9777 (0.4506 - 54.993 7) | 0,1904 | 0,0383908 3 | 311 [180;NR] vs. 48 [NR;NR] | 10.4881 (0.6555 - 167.810 4) | 0,0966 |

| Locus | p-value logrank OS | Median OS (Low expression vs. High expression) | HR OS | p-value logrank PFS | Median PFS (Low expression vs High expression) | HRPFS | Locus | p-value logrank OS |
|---|---|---|---|---|---|---|---|---|
| hsa-SYTL2_0003 | 0,0021786 09 | 58 [28;NR] vs. 869 [563;NR] | 0.0678 (0.0073 0.6276) | 0,0178 | 0,0104923 34 | 59 [16;NR] vs 468 [311;NR] | 0.1381 (0.0242 - 0.7873) | 0,0258 |
| hsa-TRIM22_000 2 | 0,0325546 57 | 79 [35;NR] vs. 869 [563;NR] | 0.1879 (0.0348 - 1.0146) | 0,052 | 0,0137839 07 | 59 [48;NR] vs 551 [231;NR] | 0.1625 (0.0319 - 0.8278) | 0,0287 |
| hsa-UTRN_0056 | 0,0011419 88 | 35 [28;NR] vs. 869 [490;NR] | 0.0568 (0.0057 - 0.5675) | 0,0146 | 0,0034332 89 | 52 [16;NR] vs 397 [231;NR] | 0.0709 (0.0072 - 0.7017) | 0,0236 |

The 13 genes out of the 23 that imply association with clinical benefit in a statistically significant manner (< 0.05) are shown below in **Table 3** and are also, both individually and in any of their combinations, biomarkers of disease prognosis in patients with metastatic cutaneous melanoma treated with nivolumab.

**Table 3:** List of circRNA loci from Table 1 prognostic markers of metastatic cutaneous melanoma in human subjects, measured as overall survival (OS) and/or disease-free progression (FPS).

| Locus | |
|---|---|
| hsa-CD74_0005 | hsa-PARP14_0007 |
| hsa-CDR1_0001 | hsa-PTPRC_0003 |
| hsa-ERBB3_0002 | hsa-SOX6_0020 |
| hsa-HLA-DRB1_0004 | hsa-SYTL2_0003 |
| hsa-HLA-DRB1_0005 | hsa-TRIM22_0002 |
| hsa-IFI30_0001 | hsa-UTRN_0056 |
| hsa-NRCAM_0112 | |

The corresponding Kaplan-Meier curves are shown in **Figures 7** and **8.** The hazard ratio analysis is shown in **Figures 9** and **10.**

In another embodiment of the invention, the expression profile is constituted by the 13 loci in **Table 3,** either individually or in any of their combinations, as prognostic markers of metastatic cutaneous melanoma in human subjects.

METHODS OF THE INVENTION

Another aspect of the invention relates to an *in vitro* method for predicting the response of a human subject to anti-PD1 and/or anti PD-L1 immune checkpoint inhibition immunotherapy, hereinafter first method of the invention, wherein the subject suffers from cancer, and wherein, using a biological sample from the human subject, the expression levels of the loci in Table 1 are determined *in vitro* individually, simultaneously or in any combination thereof, and wherein the result is indicative of a positive response if the expression levels of the loci highlighted in gray in **Table 1 are** overexpressed, while the genes in white in **Table 1** are underexpressed.

In this regard, overexpressed is preferably defined as an expression level 1.5-fold higher than the expression in the non-responder group, and underexpressed as 1.5-fold lower than the expression in the non-responder group.

More preferably, response refers to progression in the first 3 months of treatment in the case of non-responders, or complete response, partial response, stable disease or continuation of treatment for at least 1 year in the case of responders.

Another aspect of the present invention relates to a prognostic method that is performed in *vitro* using a biological sample originating from the human subject, and wherein at the time of sampling the human subject, the human subject has not yet been treated with anti-PD1 and/or anti-PD-L1 immune checkpoint inhibition immunotherapy. This prognostic method is carried out by *in vitro* determination of the expression levels of all the loci in **Table 1** simultaneously or of the 13 individual loci in **Table 3** or any combination thereof.

Patient classification is based on whether the expression value of the locus or loci used is greater or less than the median expression value of each survival group, and the result is indicative of positive prognosis if the Risk Ratio is < 1 and the result is indicative of negative prognosis if the Risk Ratio is > 1.

The outcome for prognostic assessment may refer to overall survival and/or progression-free survival.

In the context of the present invention, "Response" refers to the clinical outcome of the subject. "Response" may be expressed as overall survival or progression-free survival. Survival of cancer patients is generally adequately expressed by Kaplan-Meier curves, named after Edward L. Kaplan and Paul Meier, who first described them. The Kaplan-Meier estimator is also known as the product limit estimator. It is used to estimate the survival function from lifetime data. A plot of the Kaplan-Meier estimate of the survival function is a series of horizontal steps of decreasing magnitude that, when a large enough sample is taken, approximates the true survival function for that population. The value of the survival function between successively different sampled observations is assumed to be constant. With respect to the present invention, the Kaplan-Meier estimator can be used to measure the fraction of patients who live for a certain time after the start of chemotherapy and/or radiotherapy. The predicted clinical outcome may be survival (overall/progression-free) in months/years from the time the sample was taken. It may be survival over a specified period since sampling, such as six months or more, one year or more, two years or more, three years or more, four years or more, five years or more, six years or more. In each case, "survival" may refer to "overall survival" or "progression-free survival".

Thus, in one embodiment of the invention, the answer is the clinical outcome, which is "overall survival" (OS). "Overall survival" denotes the chances of a patient being kept alive for a group of individuals suffering from cancer. The decisive question is whether the individual is alive or dead at any given time.

*In vitro* determination of expression levels is performed by any of the techniques known in the prior art. Preferably, the techniques are selected from the list consisting of a gene profiling method, such as a microarray, or a next generation sequencing panel and/or a method comprising PCR, such as real-time PCR; and/or Northern Blot. and/or an immunohistochemistry method; and/or an ELISA-based method. RQ-PCR is a sensitive and reproducible gene expression quantification technique that can be used particularly for profiling mRNA expression in cells and tissues. Any method can be used to evaluate RT-PCR results, and preferably the ΔΔCt method (Ct = cycle threshold values). The ΔΔΔCt method will involve a control sample and a treatment sample. For each sample, a target loci and an endogenous control gene (as described below) are included for PCR amplification from aliquots (typically serially diluted). Typically, several replicates are used for each diluted concentration to obtain amplification efficiency. PCR amplification efficiency can be defined as a percentage of amplification (from 0 to 1). During the PCR reaction, software typically measures for each sample the cycle number at which the fluorescence (PCR amplification indicator) crosses an arbitrary line, the threshold. This crossover point is the Ct value. More dilute samples will cross at later Ct values. To quantify mRNA gene expression, the Ct of an RNA or DNA of the mRNA gene of interest is divided by the Ct of the endogenous control nucleic acid, such as non-tumor tissue, to normalize for variation in RNA quantity and quality between different samples. This normalization procedure is commonly referred to as the ΔΔΔCt method (Schefe et al., 2006, J. Mol. Med. 84: 901-10). Calculations of ΔΔΔCt express data in the context of the test sample (here: mRNA) versus the calibrator (endogenous control). If the ΔΔΔCt calculation is positive (e.g. +2.0), then: 2-ΔΔΔCt = 2-(2.0) = 0.25. The target quantity, normalized to an endogenous reference and relative to a calibrator, is given by: 2-ΔΔΔCt. Details of the method of calculating ΔΔΔCt can be found in: Applied Biosystems user Bulletin No. 2 (P/N 4303859). To technically validate the differentially expressed (DE) genes, the inventors employed real-time quantitative PCR (RQ-PCR) to assess gene expression of selected loci from our signature of 23 loci. Regardless of the method used to determine the response (RQ-PCR, immunohistochemistry, ELISA-based method, etc.), in the context of the present invention, we first establish a relative expression of a selection of patients who are controls for high or low expression of the genes tested against the ACTB clearance gene. This is followed by a Pearson correlation between the gene expression values generated by RNA-seq and those generated by RQ-PCR. In our case, the expression was technically validated with an overall correlation coefficient of 0.7 (p<0.001). In the context of the present invention, the myeloid and lymphocytic context of 16 melanomas were investigated in formalin-fixed, paraffin-embedded (FFPE) tissue samples. Two complementary multiplex panels were used to allow simultaneous examination of several cell markers. A random tree algorithm classifier was trained separately for each cell marker by an experienced pathologist (CEA) annotating tumor regions. Interactive feedback on cell classification performance is provided during training in the form of a marker image, which significantly improves the accuracy of machine learning-based phenotyping (PMID: 29203879, PMID: 32591586). All phenotyping and subsequent quantifications were performed without knowledge of sample identity. Cells near the edge of the images were removed to reduce the risk of artifacts. In the context of the present invention, non-limiting illustrative examples of a biological sample include different types of tissue samples, as well as biological fluids, such as blood, serum, plasma, cerebrospinal fluid, peritoneal fluid, feces. Preferably, such samples are tissue samples and, more preferably, such tissue samples originate from tumor tissue of the individual whose response is to be predicted, and may originate from biopsies.

The cancer could be any immunogenic type of cancer that is susceptible to the clinical benefit of immunotherapy. In another preferred embodiment of the invention, the cancerous disease as defined in any of the methods of the invention is melanoma, nonsmall cell lung cancer, head and neck cancer, prostate cancer, breast cancer or combinations thereof. The prognosis depends on the stage of the cancer and, in this regard, it is important to find good prognostic markers for survival after treatment for this specific disease and thus the usefulness of the biomarkers of the present invention in the prognosis of this disease. More preferably, the cancer is melanoma and, more preferably, metastatic cutaneous melanoma.

In another preferred embodiment, the anti-PD1 treatment is an anti-PD1 antibody, most preferably the anti-PD1 antibody being selected from pembrolizumab (Keytruda), cemiplimab (Libtayo) and/or nivolumab (Opdivo), and most preferably is nivolumab.

In another preferred embodiment, the anti-PD1 treatment is an anti PD-L1 antibody, most preferably anti PD-L1 antibody selected from the list consisting of: atezolizumab (Tecentriq), avelumab (Bavencio), durvalumab (Imfinzi) or combinations thereof.

Another aspect of the present invention relates to any one of the methods of the invention, wherein the method is a drug response predictive method that is performed *in vitro* using a biological sample from the human subject, and wherein at the time of sampling the human subject, the human subject has not yet been treated with anti-PD1 and/or anti-PD-L1 immune checkpoint inhibition immunotherapy. The outcome for response assessment is the clinical response using the RECIST criteria at a specific time point after treatment initiation. This method of response prediction is performed *by in vitro* determination of the expression levels of the loci in **Table 1** simultaneously or of the individual loci or any of their combinations.

Another aspect of the invention relates to a pharmaceutical composition comprising anti-PD1 and/or anti-PD-L1 immune checkpoint inhibition immunotherapy for the treatment of a human subject of a group identifiable by any of the methods of the invention.

The term "subject", as used in the description, refers to animals, preferably mammals and more preferably humans. The preferred subject is a human subject, and is not intended to be limiting in any respect; it may be of any age, sex or physical condition.

The methods of the present invention can be applied with samples of individuals of either sex, i.e., male or female, and of any age. The profile determined by the present invention is predictive and prognostic.

KIT OR DEVICE OF THE INVENTION

The present invention also provides a kit or device suitable for implementing the methods of the invention. The kit comprises at least one or more oligonucleotides capable of hybridizing to RNAs of the 23 loci of Table 1. Preferably, it allows detecting the expression level of all the genes of Table 1, simultaneously.

The kit or device is based on the predictive power of the method of the present invention. In the particular case of the kit, the reference value indicative of non-response (and/or a reference value indicative of response) can be provided with the kit. With the aid of the kit, the expression of each target gene can be calculated, i.e., relative to the endogenous control samples exemplified above. Thus, the endogenous control may also be included within the kit.

The kit can further include, without any limitation, buffers, agents to prevent contamination, protein degradation inhibitors, etc. Thus, the kit may include all necessary supports and receptacles for implementation and optimization. Preferably, the kit further comprises instructions for carrying out any of the methods of the invention.

In particular embodiments, the kit is selected from (a) a kit suitable for PCR (b) a kit suitable for microarray analysis, (c) a kit suitable for next generation sequencing. Regarding (a) a kit suitable for PCR, this PCR is typically a real-time quantitative PCR (RQ- PCR), a sensitive and reproducible gene expression quantification technique. In one embodiment, the kit comprises a microarray. An RNA microarray is an array on a solid substrate (usually a glass slide or a silicon thin film cell) that analyzes large amounts of different RNAs that are detectable through specific probes immobilized in spots on the solid substrate. Each spot contains a specific nucleic acid sequence, typically a DNA sequence, known as a probe (or reporter). While the number of spots is not limited as such, there is a preferred embodiment in which the microarray is adapted to the methods of the invention. In one embodiment, such a customized microarray comprises fifty dots or less, such as thirty dots or less, including twenty dots or less. In another embodiment of the invention, the kit comprises a series of capture probes for specific genes that are hybridized in suspension and subsequently amplified by PCR and sequenced on a low throughput sequencer since the total number of reads needed for targeted sequencing is low; therefore, it can be implemented in routine clinical laboratories.

The kit can be used and the use is not particularly limited, although use in the method of the invention in any of its embodiments is preferred. The kit can also be automated or can be incorporated into devices capable of carrying out the methods of the invention automatically.

Another aspect of the invention relates to computer-readable storage media comprising program instructions capable of causing a computer to perform the steps of any of the methods of the invention. The invention also extends to computer programs adapted to enable any processing means to carry out the methods of the invention. Such programs may take the form of source code, object code, an intermediate source of code and object code, for example, in partially compiled form, or in any other form suitable for use in implementing the processes according to the invention. The computer programs also encompass cloud applications based on this procedure. In particular, the invention encompasses computer programs arranged on or within a carrier. The carrier may be any entity or device capable of supporting the program. Where the program is embodied in a signal that can be carried directly by a cable or other device or medium, the carrier may comprise such a cable or other device or medium. As a variant, the carrier could be an integrated circuit in which the program is embedded, the integrated circuit being adapted to execute, or to be used in the execution of, the corresponding processes. For example, the programs could be embedded in a storage medium, such as a ROM, a CD ROM or a semiconductor ROM, a USB memory or a magnetic recording medium, e.g., a floppy disk or a hard disk. Alternatively, the programs could be supported by a transmittable carrier signal. For example, it could be an electrical or optical signal that could be carried via an electrical or optical cable, by radio or by any other means. The invention also extends to computer programs adapted for any processing means to carry out the methods of the invention. Such programs may take the form of source code, object code, an intermediate source of code and object code, for example, in partially compiled form, or in any other form suitable for use in implementing the processes according to the invention. Software also encompasses cloud applications based on this procedure. Thus, another aspect of the invention relates to a computer-readable storage medium comprising program instructions capable of causing a computer to carry out the steps of any of the methods of the invention. Another aspect of the invention relates to a transmittable signal comprising program instructions capable of causing a computer to carry out the steps of any of the methods of the invention.

In the context of the present invention, the terms "subject", "patient" or "individual" are used herein interchangeably to refer to all animals classified as mammals and include, but are not limited to, domestic and farm animals, primates and humans, e.g., humans, non-human primates, cows, horses, pigs, sheep, goats, dogs, cats or rodents. Preferably, the subject is a male or female human being of any age or breed.

Throughout the description and claims, the word "comprises" and variants thereof are not intended to exclude other technical features, complements, components or steps. To those skilled in the art, other objects, advantages and features of the invention will be understood in part from the description and in part from the practice of the invention. The following examples and drawings are provided by way of illustration and are not intended to limit the present invention.

## DESCRIPTION OF THE FIGURES

**Figure 1.** Association of circRNA risk score based on combined expression levels with overall survival in the cutaneous melanoma specimens analyzed. Kaplan-Meier analysis of overall survival in 12 metastatic cutaneous melanomas treated with nivolumab.

**Figure 2.** Association of circRNA risk score based on combined expression levels with progression-free survival in the cutaneous melanoma specimens analyzed. Kaplan-Meier analysis of overall survival in 12 metastatic cutaneous melanomas treated with nivolumab.

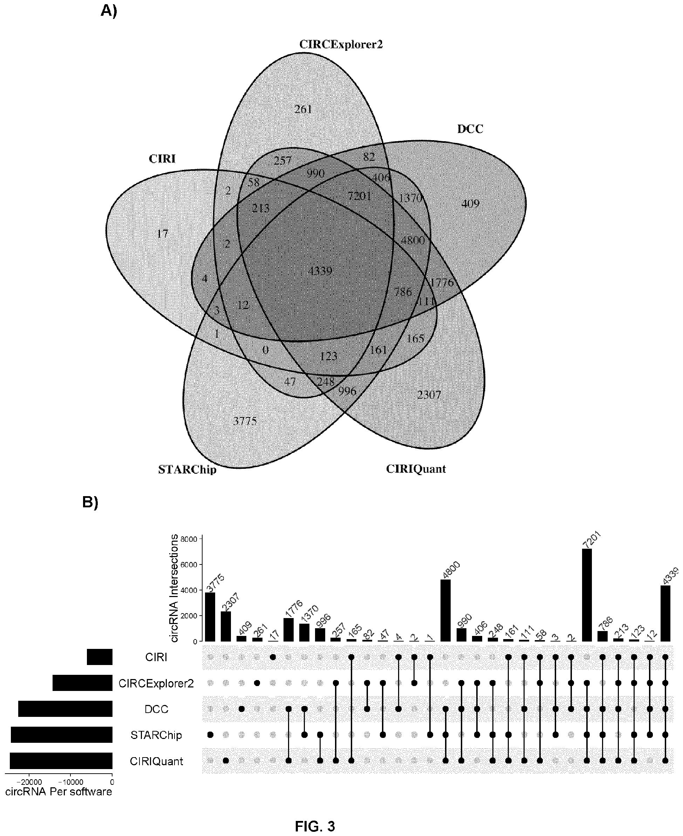

**Figure 3**. (A) Venn diagram showing the common circRNAs identified by the prediction algorithms tested. 4339 circRNA were identified by the five algorithms used to map and identify circRNA, (B) Altered graph showing the number of circRNA among the different algorithms used.

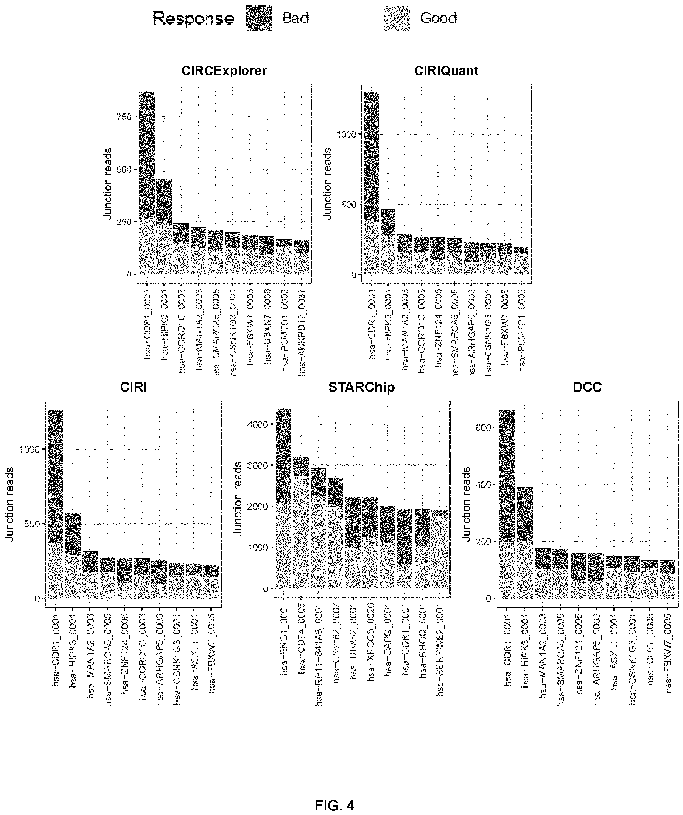

**Figure 4.** Top 10 circRNA identified by the different algorithms used. The figure represents the total number of counts in good and bad responders for the top 10 circRNAs.

**Figure 5.** Volcano plot of differential circRNA gene expression between good and poor responders. By setting multiple difference threshold (fold-change) higher than 1.5 and a p-value lower than 0.1, 23 aberrantly expressed circRNAs are observed.

**Figure 6.** Heat map representing the expression of the different differentially expressed circRNAs In the figure the adjusted p-value is less than 0.1. A pattern of expression in some subsets of genes between good and poor responders is clearly visible.

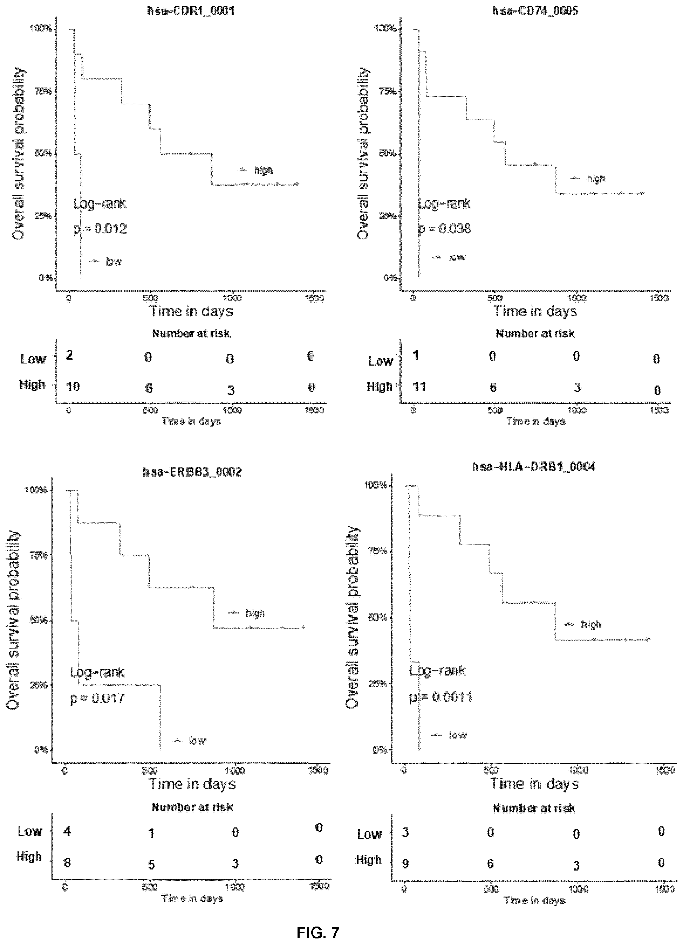

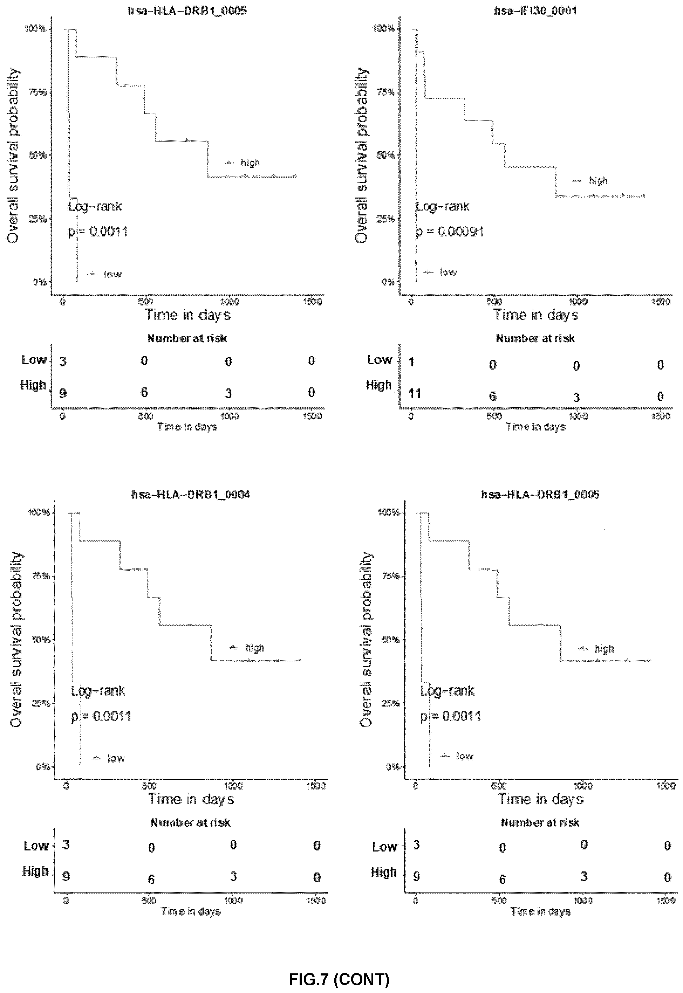

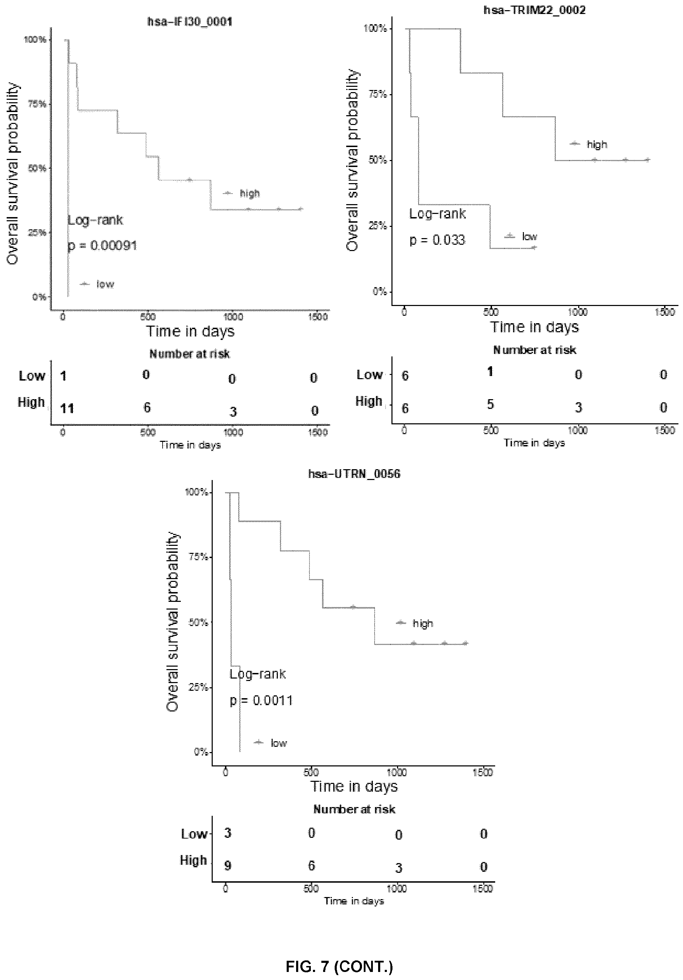

**Figure 7.** Association of the risk score of the 11 prognostically associated circRNAs based on individual expression levels with overall survival in the cutaneous melanoma specimens analyzed. Kaplan-Meier analysis of overall survival in 12 metastatic cutaneous melanomas treated with nivolumab.

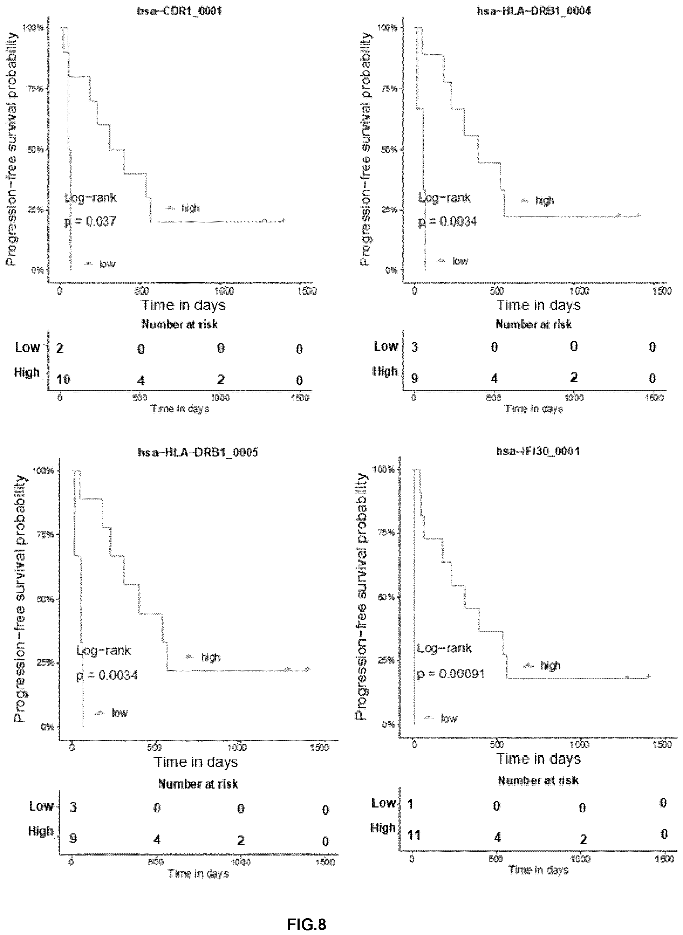

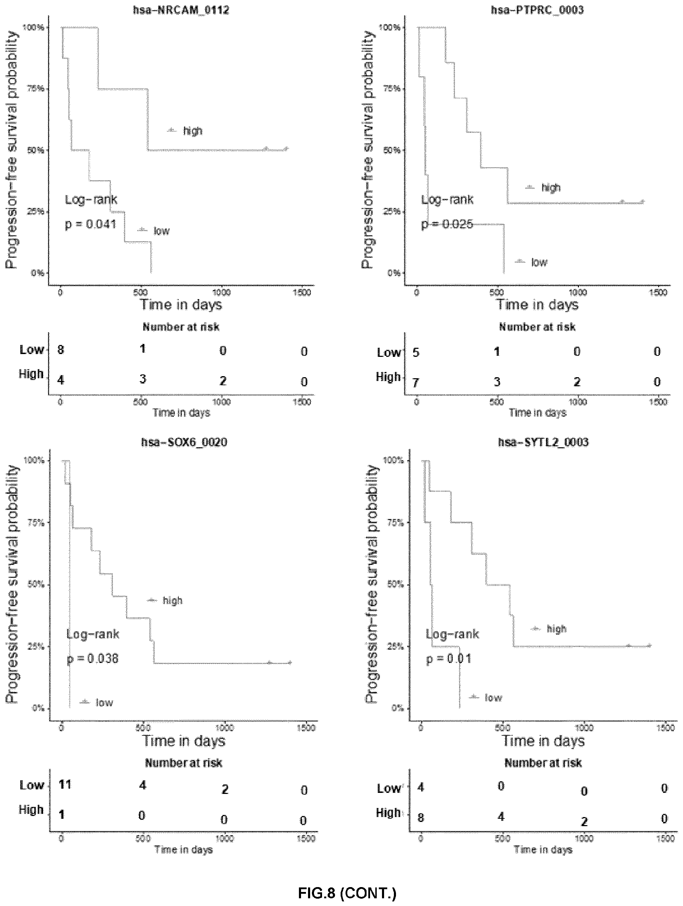

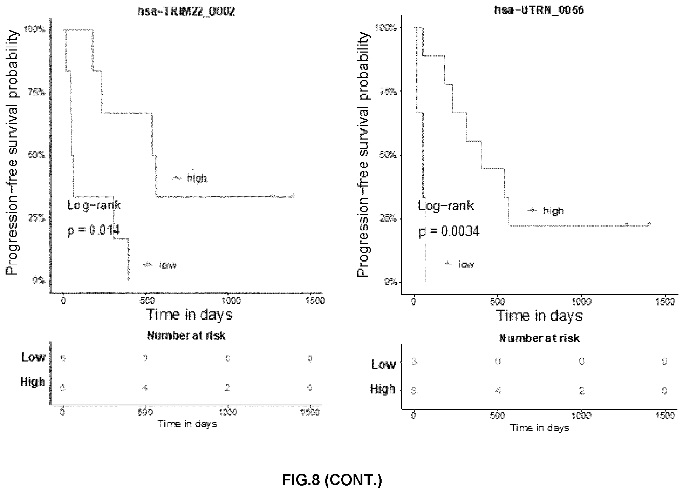

**Figure 8.** Association of the risk score of the 10 prognostically associated circRNAs based on individual expression levels with progression-free survival in the cutaneous melanoma samples analyzed. Kaplan-Meier analysis of overall survival in 12 metastatic cutaneous melanomas treated with nivolumab.

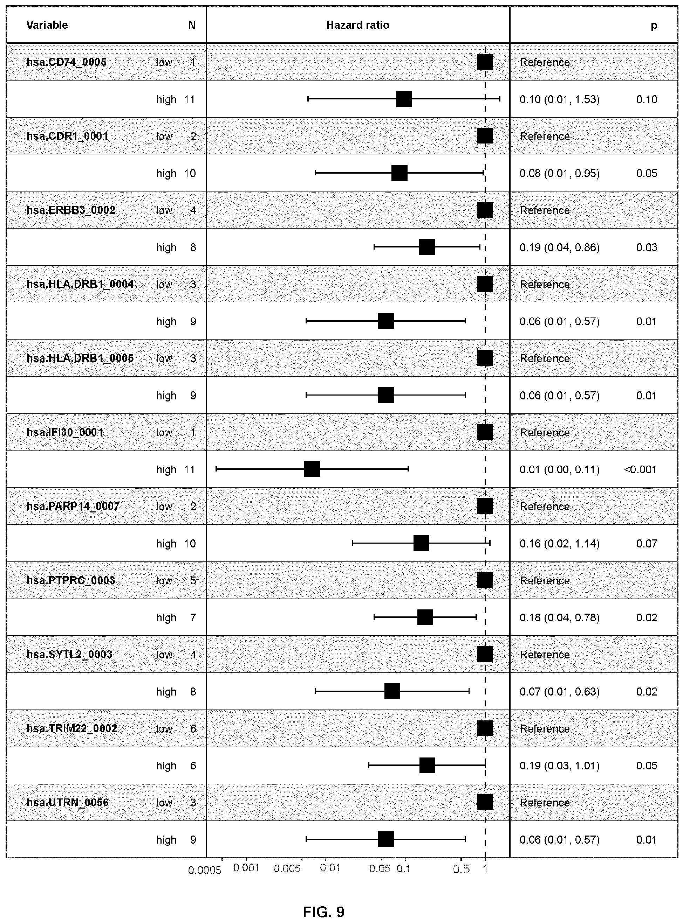

**Figure 9.** Univariate hazard ratio analysis of each of the prognostic circRNAs with respect to overall survival in the 12 analyzed cutaneous melanoma samples corresponding to patients with metastatic cutaneous melanoma treated with nivolumab.

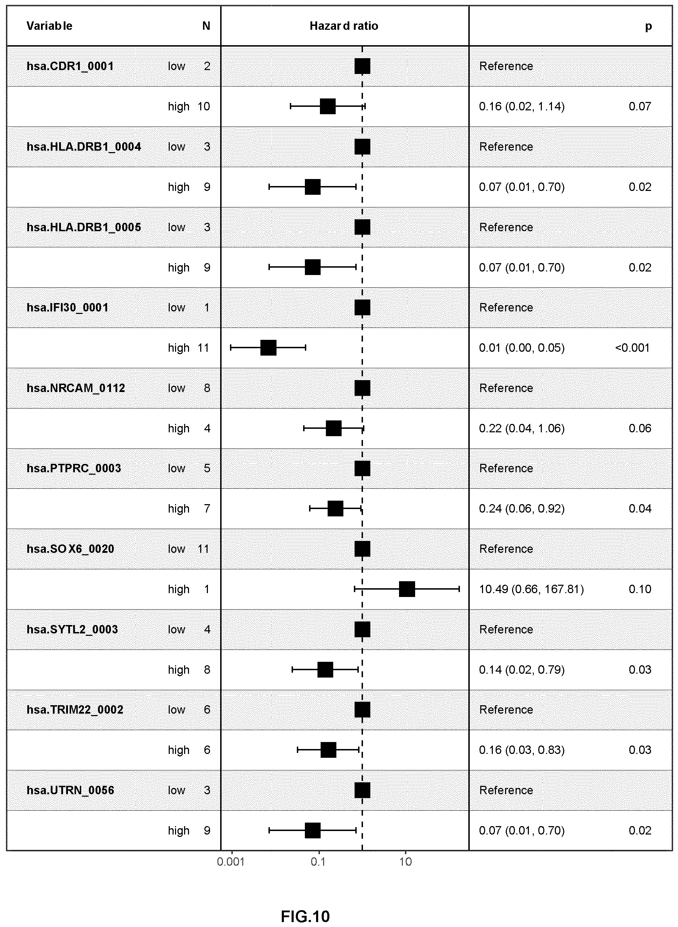

**Figure 10.** Univariate hazard ratio analysis of each of the prognostic circRNAs with respect to progression-free survival in the 12 analyzed cutaneous melanoma samples corresponding to patients with metastatic cutaneous melanoma treated with nivolumab.

## EXAMPLES OF THE INVENTION

The following specific examples provided in this patent document serve to illustrate the nature of the present invention. These examples are included for illustrative purposes only and are not to be construed as limiting the invention claimed herein. Thus, the examples described above illustrate the invention without limiting the scope of the invention.

**Subjects**

A total of 12 patients with metastatic cutaneous melanoma treated with nivolumab donated formalin-fixed paraffin-embedded (FFPE) tissue biopsy samples that were collected in a pre-treatment state at the Hospital Regional de Málaga and Hospital Universitario Virgen de la Victoria (Málaga). The study follows the Declaration of Helsinki and is vetted by the Ethics Committee of Malaga.

**Nucleic acid extraction**

The specific tumor area in FFPE melanoma specimens was predefined by a pathologist. Two to four 10 $\mu$m slides were dissected for nucleic acid extraction using the HM 340E microtome (Thermo Scientific). RNA was extracted with the RNeasy FFPE kit (Qiagen).

**Next generation sequencing**

RNA-Seq libraries were prepared with TruSeq Stranded Total RNA Gold (Illumina; Ref. 20020598) and indexed by IDT for Illumina: TruSeq RNA UD indices (Illumina). Library concentration was determined with the Qubit dsDNA BR kit and size distribution was examined with Agilent Bioanalyzer. Paired-end reads (75 bp $\times$ 2) were acquired from the Illumina NextSeq 550 platform according to the corresponding protocol.

**Real-time PCR validation**

Expression levels of CDR1-AS, the most frequent circRNAs, were verified by the predesigned qRT-PCR taqman probe used in all samples (Hs05016408_s1).

**CirRNA detection**

Fastq data quality control of the paired end reads was performed with FastQC. Fastq files were trimmed with a Q30 cutoff. We evaluated five different pipelines to identify and quantify circRNA reading circRNAs. CIRI, CIRIExplorer2, DCC, STARchip and CIRIQUANT were used and compared. The CircBase database and the cir-c2Trait disease database were used to annotate identified circRNAs. To obtain high-confidence circRNAs, we used a minimum filter cutoff of 2 binding reads in at least 2 samples and in at least 3 software (validation strategy), which allowed for a minimum number of back splicing reads (BSJ) per circRNA. This criterion resulted in 4339 unique circRNAs across all samples, and we used these high-confidence circRNAs for all analyses performed in this study. With direct splice junction reads (FSJ) and back splice junction reads (BSJ), we used the following formula: 2*bsj/(2*bsj+fsj) to calculate the ratio of circular to linear transcripts.

**Analysis of differential expressions**

The DESq2 battery of total mapped reads was used to perform high-confidence circRNA differential expression (DE). Differential expression analysis was based on negative binomial generalized linear models and threshold values were adjusted with a p-adjusted value of $p < 0.1$ and an absolute value of log2 (fold change) > 1.5. In differential expression (DE) analysis the total linear mapped linear read counts were used for size factor estimation.

**Statistics**

Statistical analysis graphs and tables were performed using R 4.0.2. The Venn Diagram R package was used to create Venn diagrams. The ComplexHeatmap R package (Gu, Eils, & Schlesner, 2016) was used to create the heat maps. The package ggplot2 (Wickham, 2009) was used to create the subsequent diagrams and plots.
In the survival analysis, Kaplan-Meier (KM) and Logrank tests were used to test the difference between groups.
For the full signature study, the risk score for each patient was estimated using the method described previously (Wang et al., 2021). Based on circRNA expression value weighted by regression coefficients in univariate cox regression analysis.

$$\text{Risk Score (RS)} = \sum_{i=1}^{N} (\text{Expression}_i * \text{Coefficient}_i)$$

N is the number of DE (Differentially Expressed) circRNA, Expression-i represents the normalized expression value and Coefficient-i is the Cox regression coefficient in the univariate model.

For the study of individual genes, the risk score was performed by calculating the Hazard Ratio (HR). The HR is calculated using the Cox univariate model of normalized expression values.

The classification of the patient under study is made according to whether the expression value of the locus or loci used is higher or lower than the median expression value of each survival group.

The result is indicative of positive prognosis if the Risk Ratio is < 1 and the result is indicative of negative prognosis if the Risk Ratio is > 1.

References

- Aldea, M. et al. (2021) 'Overcoming Resistance to Tumor-Targeted and Immune-Targeted Therapies', Cancer Discovery. American Association for Cancer Research, 11(4), pp. 874-899. doi: 10.1158/2159-8290.CD-20-1638.

- Filipovic, A., Miller, G. and Bolen, J. (2020) 'Progress Toward Identifying Exact Proxies for Predicting Response to Immunotherapies', Frontiers in Cell and Developmental Biology. Frontiers, p. 155. doi: 10.3389/fcell.2020.00155.

- Gu, Z., Eils, R. and Schlesner, M. (2016) 'Complex heatmaps reveal patterns and correlations in multidimensional genomic data', Bioinformatics. Bioinformatics, 32(18), pp. 2847-2849. doi: 10.1093/bioinformatics/btw313.

- Montico, B. et al. (2021) 'The pleiotropic role of circular and long noncoding RNAs in cutaneous melanoma', Molecular Oncology. John Wiley & Sons, Ltd. doi: 10.1002/1878-0261.13034.

- QIAGEN Inc (2016) Ingenuity Pathway Analysis (IPA). Available at: hftps://www.qiagen.com/us/products/discovery-and-translational-research/next-generation-sequencing/informatics-and-data/interpretation-content-databases/ingenuity-pathway-analysis/ (Accessed: 30 September 2021).

- Tay, Y., Rinn, J. and Pandolfi, P. P. (2014) 'The multilayered complexity of ceRNA crosstalk and competition', Nature. Nature, pp. 344-352. doi: 10.1038/nature12986.

- Wang, W. et al. (2021) 'RNA sequencing reveals the expression profiles of circRNA and identifies a four-circRNA signature acts as a prognostic marker in esophageal squamous cell carcinoma', Cancer Cell International. BioMed Central Ltd, 21(1), pp. 1-14. doi: 10.1186/S12935-021-01852-9/FIGURES/6.

**Claims**

1. Use of the loci in Table 1, individually or any of their combinations, to predict response to anti-PD1 and/or anti-PD-L1 immune checkpoint inhibition immunotherapy in the treatment of metastatic cutaneous melanoma.

2. Use according to claim 1, wherein the use of the loci of Table 1 is simultaneous.

3. A biomarker expression profile suitable for predicting response to anti-PD1 and/or anti-PD-L1 immune checkpoint inhibition immunotherapy in the treatment of metastatic cutaneous melanoma comprising the expression levels of the loci in Table 1 individually or in any of their combinations.

4. *In vitro* method of obtaining data useful for predicting or prognosticating the response of a human subject to anti-PD1 and/or anti-PD-L1 immune checkpoint inhibition immunotherapy, wherein the subject has metastatic cutaneous melanoma, from a biological sample previously isolated from the individual, comprising:

    a) quantifying the expression levels of the loci in Table 1 separately or in any of their combinations.

5. The *in vitro* method of obtaining useful data according to claim 4 further comprising: b) comparing the quantities obtained in step (a) with a reference quantity.

6. *In vitro* method for predicting the response of a human subject to anti-PD1 and/or anti-PD-L1 immune checkpoint inhibition immunotherapy, wherein the subject has metastatic cutaneous melanoma, comprising the method of obtaining useful data according to the preceding claim, wherein the result is indicative of a positive response if the expression levels the loci highlighted in gray in Table 1 are overexpressed while those highlighted in white are underexpressed.

7. The method according to claim 6 wherein overexpressed is defined as an expression level 1.5 times higher than the expression in the non-responder group, and underexpressed as 1.5 times lower than the expression in the non-responder group.

8. The method according to any one of claims 6-7, wherein the term response refers to progression in the first 3 months of treatment in the case of non-responders, or complete response, partial response, stable disease or continuation of treatment for at least 1 year in the case of responders.

9. Simultaneous use of all the loci in Table 1 or the loci in Table 3, individually or in any of their combinations, to predict the survival of a human subject with metastatic cutaneous melanoma, prior to treatment with anti-PD1 and/or anti-PD-L1 inhibitors.

10. A biomarker expression profile suitable for predicting the survival of a human subject with metastatic cutaneous melanoma, prior to treatment with anti-PD1 and/or anti-PD-L1 inhibitors, comprising the expression levels of all the loci in Table 1, or any of the labeled loci in Table 3 individually or any of their combinations.

11. *In vitro* method of obtaining data useful for predicting the survival of a human subject with metastatic cutaneous melanoma, from a biological sample previously isolated from the individual, comprising:

a) quantifying the expression levels of all the loci in Table 1 simultaneously or of any of the loci in Table 3 individually or any combination thereof.

12. The *in vitro* method of obtaining useful data according to claim 11 further comprising: b) comparing the quantities obtained in step (a) with a reference quantity.

13. An *in vitro* method for predicting the survival of a human subject suffering from metastatic cutaneous melanoma, prior to treatment with anti-PD1 and/or anti-PD-L1 inhibitors, comprising the method of obtaining useful data according to the preceding claim, wherein the classification of the patient is based on whether the expression value of the locus or loci used is greater or less than the median expression value of each survival group, and the result is indicative of positive prognosis if the Risk Ratio is < 1 and the result is indicative of negative prognosis if the Risk Ratio is > 1.

14. The method according to any one of claims 11-13, wherein the term survival refers to overall survival and/or disease-free progression.

15. The method according to claims 4 to 8 and 11 to 14 wherein the *in vitro* determination of the expression levels of the loci is carried out by a gene profiling method, such as a microarray, or a massive sequencing panel and/or a method comprising PCR, such as real-time PCR; and/or Northern Blot. and/or an immunohistochemistry method; and/or an ELISA-based method.

16. The method according to claims 4 to 8 and 11 to 15, wherein the protein or mRNA encoded by the loci selected from Table 1 is used as an indicator.

17. The method according to any one of claims 4 to 8 and 11 to 16, wherein the biological sample is fresh tissue, paraffin-embedded tissue or RNA extracted from a tissue of a patient with metastatic cutaneous melanoma.

18. The method according to any one of claims 4 to 8 and 11 to 17, wherein the anti-PD1 immune checkpoint inhibition immunotherapy is selected from the list consisting of: pembrolizumab, nivolumab, cemiplimab or combinations thereof.

19. The method according to any one of claims 4 to 8 and 12 to 18, wherein the anti PD-L1 immune checkpoint inhibition immunotherapy is selected from the list consisting of: atezolizumab, avelumab, durvalumab or combinations thereof.

20. A method for classifying a human subject suffering from metastatic cutaneous melanoma into one of two groups, wherein group 1 comprises subjects identifiable by the method according to any one of claims 6 - 8; and wherein group 2 represents the remaining subjects.

21. A pharmaceutical composition comprising anti-PD1 and/or anti PD-L1 immune checkpoint inhibition immunotherapy for treating a human subject identifiable as belonging to group 1 according to the method of claim 20.

22. A kit or device suitable for performing the methods according to any one of claims 4 to 8 and 11 to 20, comprising oligonucleotides capable of hybridizing to RNAs of the loci of Table 1, individually or in any combination thereof.

FIG. 1

FIG. 2

**A)**

**B)**

FIG. 3

FIG. 4

FIG. 5

FIG. 6

FIG. 7

FIG.7 (CONT)

FIG. 7 (CONT.)

FIG.8

FIG.8 (CONT.)

FIG.8 (CONT.)

| Variable | | N | Hazard ratio | | p |
|---|---|---|---|---|---|
| **hsa.CD74_0005** | low | 1 | | Reference | |
| | high | 11 | | 0.10 (0.01, 1.53) | 0.10 |
| **hsa.CDR1_0001** | low | 2 | | Reference | |
| | high | 10 | | 0.08 (0.01, 0.95) | 0.05 |
| **hsa.ERBB3_0002** | low | 4 | | Reference | |
| | high | 8 | | 0.19 (0.04, 0.86) | 0.03 |
| **hsa.HLA.DRB1_0004** | low | 3 | | Reference | |
| | high | 9 | | 0.06 (0.01, 0.57) | 0.01 |
| **hsa.HLA.DRB1_0005** | low | 3 | | Reference | |
| | high | 9 | | 0.06 (0.01, 0.57) | 0.01 |
| **hsa.IFI30_0001** | low | 1 | | Reference | |
| | high | 11 | | 0.01 (0.00, 0.11) | <0.001 |
| **hsa.PARP14_0007** | low | 2 | | Reference | |
| | high | 10 | | 0.16 (0.02, 1.14) | 0.07 |
| **hsa.PTPRC_0003** | low | 5 | | Reference | |
| | high | 7 | | 0.18 (0.04, 0.78) | 0.02 |
| **hsa.SYTL2_0003** | low | 4 | | Reference | |
| | high | 8 | | 0.07 (0.01, 0.63) | 0.02 |
| **hsa.TRIM22_0002** | low | 6 | | Reference | |
| | high | 6 | | 0.19 (0.03, 1.01) | 0.05 |
| **hsa.UTRN_0056** | low | 3 | | Reference | |
| | high | 9 | | 0.06 (0.01, 0.57) | 0.01 |

0.0005  0.001  0.005  0.01  0.05  0.1  0.5  1

FIG. 9

| Variable | | N | Hazard ratio | | p |
|---|---|---|---|---|---|
| **hsa.CDR1_0001** | low | 2 | | Reference | |
| | high | 10 | | 0.16 (0.02, 1.14) | 0.07 |
| **hsa.HLA.DRB1_0004** | low | 3 | | Reference | |
| | high | 9 | | 0.07 (0.01, 0.70) | 0.02 |
| **hsa.HLA.DRB1_0005** | low | 3 | | Reference | |
| | high | 9 | | 0.07 (0.01, 0.70) | 0.02 |
| **hsa.IFI30_0001** | low | 1 | | Reference | |
| | high | 11 | | 0.01 (0.00, 0.05) | <0.001 |
| **hsa.NRCAM_0112** | low | 8 | | Reference | |
| | high | 4 | | 0.22 (0.04, 1.06) | 0.06 |
| **hsa.PTPRC_0003** | low | 5 | | Reference | |
| | high | 7 | | 0.24 (0.06, 0.92) | 0.04 |
| **hsa.SOX6_0020** | low | 11 | | Reference | |
| | high | 1 | | 10.49 (0.66, 167.81) | 0.10 |
| **hsa.SYTL2_0003** | low | 4 | | Reference | |
| | high | 8 | | 0.14 (0.02, 0.79) | 0.03 |
| **hsa.TRIM22_0002** | low | 6 | | Reference | |
| | high | 6 | | 0.16 (0.03, 0.83) | 0.03 |
| **hsa.UTRN_0056** | low | 3 | | Reference | |
| | high | 9 | | 0.07 (0.01, 0.70) | 0.02 |

0.001    0.1    10

FIG.10

# INTERNATIONAL SEARCH REPORT

| | |
|---|---|
| International application No. | |
| PCT/ES2023/070341 | |

**A. CLASSIFICATION OF SUBJECT MATTER**

*C12Q1/6886* (2018.01)

According to International Patent Classification (IPC) or to both national classification and IPC

**B. FIELDS SEARCHED**

Minimum documentation searched (classification system followed by classification symbols)

C12Q

Documentation searched other than minimum documentation to the extent that such documents are included in the fields searched

Electronic data base consulted during the international search (name of data base and, where practicable, search terms used)

EPODOC, INVENES, WPI, BIOSIS, MEDLINE, EMBASE, INSPEC, NPL, INTERNET

**C. DOCUMENTS CONSIDERED TO BE RELEVANT**

| Category* | Citation of document, with indication, where appropriate, of the relevant passages | Relevant to claim No. |
|---|---|---|
| A | ONIEVA J L et al.. Evaluation of circular RNA profiling in metastatic melanoma patients treated with immune checkpoint blockade. Cancer Research 20200801 American Association For Cancer Research Inc. Nld., 01/08/2020, Vol. 80, N° 16 SUPPL, pages Abstract 281 Retrieved from <URL: https://doi.org/10.1158/1538-7445.AM2020-281>, ISSN 1538-7445 (print), <DOI: 10.1158/1538-7445.AM2020-281> the whole document. | 1-8, 15-20, 22 |
| A | ZHOU J -G et al.. Identification of 15 lncrnas signature for predicting survival benefit of advanced melanoma patients treated with anti-pd-1 monotherapy. Cells 20210501 Mdpi Che., 01/05/2021, Vol. 10, N° 5, pages Article No.: 977, ISSN 2073-4409 (electronic), <DOI: 10.3390/cells10050977 pubmed:33922038> the whole document. | 1-8, 15-20, 22 |

☒ Further documents are listed in the continuation of Box C. ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 16/10/2023 | **(07/11/2023)** |

| Name and mailing address of the ISA/ | Authorized officer |
|---|---|
| OFICINA ESPAÑOLA DE PATENTES Y MARCAS Paseo de la Castellana, 75 - 28071 Madrid (España) Facsimile No.: 91 349 53 04 | J. Vizán Arroyo Telephone No. 913498573 |

Form PCT/ISA/210 (second sheet) (July 2022)

## INTERNATIONAL SEARCH REPORT

| | International application No. |
|---|---|
| | PCT/ES2023/070341 |

| C (continuation). | DOCUMENTS CONSIDERED TO BE RELEVANT | |
|---|---|---|
| Category * | Citation of documents, with indication, where appropriate, of the relevant passages | Relevant to claim No. |
| A | XUE LING et al.. Using Immune-Related lncRNA Signature for Prognosis and Response to Immunotherapy in Cutaneous Melanoma.. International Journal Of General Medicine New Zealand 2021., 30/11/2020, Vol. 14, pages 6463 - 6475, ISSN 1178-7074 (Print), <DOI: 10.2147/IJGM.S335266 pubmed:34675614> the whole document. | 1-8, 15-20, 22 |
| A | WO 2021110927 A1 (SERVICIO ANDALUZ DE SALUD ET AL.) 10/06/2021, the whole document. | 1-8, 15-20, 22 |
| P,X | OLIVER JAVIER et al.. Association of Circular RNA and Long Non-Coding RNA Dysregulation with the Clinical Response to Immune Checkpoint Blockade in Cutaneous Metastatic Melanoma. Biomedicines Oct 2022., 30/09/2022, Vol. 10, N° 10, pages Article No.: 2419, ISSN 2227-9059(electronic), <DOI: 10.3390/biomedicines10102419> the whole the document. | 1-8, 15-20, 22 |

Form PCT/ISA/210 (continuation of second sheet) (July 2022)

**RAPPORT DE RECHERCHE INTERNATIONALE**

Demande internationale n°

PCT/ES2023/070341

| Cadre n° II | Observations - lorsqu'il a été estimé que certaines revendications ne pouvaient pas faire l'objet d'une recherche (suite du point 2 de la première feuille) |

Le rapport de recherche internationale n'a pas été établi en ce qui concerne certaines revendications conformément à l'article 17.2)a) pour les raisons suivantes :

1. ☐ Les revendications n°ˢ :
se rapportent à un objet à l'égard duquel l'administration chargée de la recherche internationale n'est pas tenue de procéder à la recherche, à savoir :

2. ☒ Les revendications n°ˢ :
parce qu'elles se rapportent à des parties de la demande internationale qui ne remplissent pas suffisamment les conditions prescrites pour qu'une recherche significative puisse être effectuée, en particulier :

The pharmaceutical composition which is the subject matter of claim 21 is not clearly defined and therefore does not comply with PCT Article 6. Said composition is characterised only by the terms that define the technical problem addressed, i.e. a composition suitable for the inhibitor immunotherapy of the anti-PD1 and/or anti-PD-L1 immune checkpoints for treating a subject with metastatic cutaneous melanoma; and not by the constitutive compounds of said composition.

3. ☐ Les revendications n°ˢ :
parce qu'elles sont des revendications dépendantes et ne sont pas rédigées conformément aux dispositions de la deuxième et de la troisième phrases de la règle 6.4.a).

| Cadre n° III | Observations - lorsqu'il y a absence d'unité de l'invention (suite du point 3 de la première feuille) |

L'administration chargée de la recherche internationale a trouvé plusieurs inventions dans la demande internationale, à savoir :

SEE SUPPLEMENTAL BOX PCT/ISA/210

1. ☐ Comme toutes les taxes additionnelles exigées ont été payées dans les délais par le déposant, le présent rapport de recherche internationale porte sur toutes les revendications pouvant faire l'objet d'une recherche.

2. ☐ Comme toutes les revendications qui se prêtent à la recherche ont pu faire l'objet de cette recherche sans effort particulier justifiant des taxes additionnelles, l'administration chargée de la recherche internationale n'a sollicité le paiement d'aucunes taxes de cette nature.

3. ☐ Comme une partie seulement des taxes additionnelles demandées a été payée dans les délais par le déposant, le présent rapport de recherche internationale ne porte que sur les revendications pour lesquelles les taxes ont été payées, à savoir les revendications n°ˢ :

4. ☒ Aucunes taxes additionnelles demandées n'ont été payées dans les délais par le déposant. En conséquence, le présent rapport de recherche internationale ne porte que sur l'invention mentionnée en premier lieu dans les revendications; elle est couverte par les revendications n°ˢ : n°ˢ:1-8, (15-20, 22) (partially)

**Remarque quant à la réserve** ☐ Les taxes additionnelles étaient accompagnées d'une réserve de la part du déposant et, le cas échéant, du paiement de la taxe de réserve.

☐ Les taxes additionnelles étaient accompagnées d'une réserve de la part du déposant mais la taxe de réserve n'a pas été payée dans le délai prescrit dans l'invitation.

☐ Le paiement des taxes additionnelles n'était assorti d'aucune réserve.

Formulaire PCT/ISA/210 (suite de la première feuille (2)) (janvier 2015)

**INTERNATIONAL SEARCH REPORT**

International application No.

PCT/ES2023/070341

The patent application does not meet the requirement of unity of invention (PCT Article 3(4)(iii) and PCT Rule 13) and includes the following inventions or groups of inventions:

Invention 1: (claims 1-8, (15-20, 22) (in part)) relates to the use of the locus hsa-ALDH1L2_0014 for predicting the response to the inhibitor immunotherapy of anti-PD1 and/or anti-PD-L1 immune checkpoints in the treatment of metastatic cutaneous melanoma. An expression profile of the biomarker hsa-ALDH1L2_0014, suitable for predicting the response to the anti-PD1 and/or anti-PD-L1 immunotherapy in the treatment of metastatic cutaneous melanoma. An in vitro method for obtaining useful data for predicting or prognosticating the response of a subject with metastatic cutaneous melanoma to anti-PD1 and/or anti-PD-L1 immunotherapy, said method comprising quantifying the expression levels of the locus hsa-ALDH1L2_0014. A kit or a device for implementing said method.

Inventions 2 to 23: (claims 1-8, (15-20, 22) (in part)) ibid for the loci from hsa-CD38_0001 to hsa-UTRN_0056 in table 1.

Invention 24: (claims 9-19, 22) (in part)) relates to the simultaneous use of the 23 loci in table 1 to predict the survival of a subject with metastatic cutaneous melanoma, prior to treatment with anti-PD1 and/or anti-PD-L1 inhibitors. An expression profile of the 23 loci in table 1 for predicting the survival of a subject with metastatic cutaneous melanoma, prior to the anti-PD1 and/or anti-PD-L1 treatment. An in vitro method for obtaining useful data for predicting the survival of a subject with metastatic cutaneous melanoma, said method comprising quantifying the expression levels of the 23 loci in table 1. A kit or a device for implementing said method.

Inventions 25 to 37: (claims 9-19, 22) (in part)) ibid for each one of the 13 loci in table 3.

Form PCT/ISA/210 (extra sheet) (January 2015)

## INTERNATIONAL SEARCH REPORT

Information on patent family members

International application No.

PCT/ES2023/070341

| Patent document cited in the search report | Publication date | Patent family member(s) | Publication date |
|---|---|---|---|
| WO2021110927 A1 | 10.06.2021 | EP4069869 A1 | 12.10.2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SCHEFE et al.** *J. Mol. Med.*, 2006, vol. 84, 901-10
- Overcoming Resistance to Tumor-Targeted and Immune-Targeted Therapies. **ALDEA, M. et al.** Cancer Discovery. American Association for Cancer Research, 2021, vol. 11, 874-899
- **FILIPOVIC, A.** ; **MILLER, G.** ; **BOLEN, J**. Progress Toward Identifying Exact Proxies for Predicting Response to Immunotherapies. *Frontiers in Cell and Developmental Biology. Frontiers*, 2020, 155
- **GU, Z.** ; **EILS, R.** ; **SCHLESNER, M.** Complex heatmaps reveal patterns and correlations in multidimensional genomic data. *Bioinformatics. Bioinformatics*, 2016, vol. 32 (18), 2847-2849

- The pleiotropic role of circular and long noncoding RNAs in cutaneous melanoma. **MONTICO, B. et al.** Molecular Oncology. John Wiley & Sons, Ltd, 2021
- *Ingenuity Pathway Analysis (IPA)*, 2016, hftps://www. qiagen.com/us/products/discovery-and-translational-research/next-generation-sequencing/informatics-and-data/interpretation-content-database-s/ingenuity-pathway-analysis
- **TAY, Y.** ; **RINN, J.** ; **PANDOLFI, P. P.** The multilayered complexity of ceRNA crosstalk and competition. *Nature. Nature*, 2014, 344-352
- RNA sequencing reveals the expression profiles of circRNA and identifies a four-circRNA signature acts as a prognostic marker in esophageal squamous cell carcinoma. **WANG, W. et al.** Cancer Cell Interna-tional. BioMed Central Ltd, 2021, vol. 21, 1-14